# EUROPEAN PATENT SPECIFICATION

(11) **EP 2 405 951 B1**
(45) Date of publication and mention of the grant of the patent: **22.11.2017**
(21) Application number: 10722152.5
(22) Date of filing: 11.03.2010
(51) Int. Cl.: A61L 15/10, A61L 15/12

(54) **NOVEL COMPOSITE MATERIALS COMPRISING A THERMOPLASTIC MATRIX POLYMER AND WOOD PARTICULES**
NEUE KOMPOSIT-MATERIALIEN AUS EINEM THERMOPLASTISCHEN MATRIXPOLYMER UND HOLZTEILCHEN
PROCÉDÉ DE PRODUCTION DE MATÉRIAUX COMPOSITES À MATRICE THERMOPLASTIQUE COMPRENANT DES PARTICULES DE BOIS

(30) Priority: 11.03.2009 FI 20095251
(43) Date of publication of application: 18.01.2012
(73) Proprietor: Onbone Oy, 00100 Helsinki (FI)
(72) Inventor: PÄRSSINEN, Antti, 00100 Helsinki (FI); LAHTINEN, Petro, 00100 Helsinki (FI)
(74) Representative: Oxley, Rachel Louise
(86) International application number: PCT/FI2010/050187
(87) International publication number: WO 2010/103188

(56) References cited:
- WO-A1-94/03211
- WO-A2-2007/035875
- WO-A2-2008/116025
- CAULFIELD D F ET AL: "Handbook of wood chemistry and wood composites, Chapter 13 (Wood Thermoplastic Composites)", 1 January 2005 (2005-01-01), HANDBOOK OF WOOD CHEMISTRY AND WOOD COMPOSITES, CRC PRESS, BOCA RATON, FLA. [U.A.], PAGE(S) 365 - 378, XP002608185, ISBN: 978-0-8493-1588-6

## Description

### BACKGROUND OF THE INVENTION

### Field of Invention

The present invention relates, in general to the field of manufacturing wood-plastic composite (WPC). In particular, present invention relates to the production of composite materials comprising wood particles and biodegradable thermoplastic polymer. More particularly, the present invention relates to a method according to the preamble of claim 1 of manufacturing novel wood-plastic composites useful as splints or casts in immobilization of a fractured body extremities.

### Description of related art

There are several orthopedic applications which comprise thermoplastic polymers, in particular polycaprolactone and finely divided wood powder or fibres and thermoplastic polymers. Primarily these structures are of the "mesh type" consisting of cellulosic fibers with an average particle size less than 0.6 mm. Thus, a mesh type casting material is presented in Published International Patent Application WO 94/03211. The material is manufactured by using a powder deposition process. The process includes mixing, with a dispensing hopper, depositing the material to the non-woven fabric followed by a pressing procedure and a heat treatment procedure at a double-band press.

Powder deposition is however suitable only for manufacturing homogenized composites with fine particles. Moreover, the double-bend press cannot gently homogenize the wood particles with rather large dimensions to an evenly distributed mixture with polymer matrix.

Another method of manufacturing medical composites is presented in the Published International Patent Application WO 2008/041215 in which one component of the structure is polycaprolactone but, like in the previous case, the material consists of many layers including spacer fabric. In addition, the splinting assembly is pre-shaped to match a fractured limb. The described manufacturing process includes both injection and compression molding. Due to large size of the wood particles, and the PCL having a very low melt flow index value, shaping of the material of the present invention by injection based systems is not practical. The pressure during processing may increase to so high levels that decomposition of wood's three dimensional structure is apparent.

Yet another alternative is presented in a U.S. Published Patent Application US2008/0262400, which concerns moldable composite splints consist of several layers, one being thermoplastic polycaprolactone. The composite material of the reference is manufactured by heat welding in a vacuum press followed by a perforating process with a turret punch press. This moldable perforated splint composite consists of a plurality of layers which can be nicely manufactured with techniques presented in reference. This technique will not make it possible to produce a composite having a uniform structure in which wood particles of granular or platy structure are thoroughly and evenly dispersed.

Methods of manufacturing wood thermoplastic composites of wood flour or wood fibers are discussed in Caulfield, D F et al.: "Handbook of wood chemistry and wood composites, Chapter 13 (Wood Thermoplastic Composite)", 1 January 2005 (2005-01-01), Handbook of Wood Chemistry and Wood Composites, CRC PRESS, Boca Raton, FLA. [U.A.], p. 365-378. The thermoplastic matrix materials listed in the reference are polyethylene, polypolylene and polyvinyl chloride.

### Summary of invention

It is an aim of the present invention to eliminate at least some of the problems of the art and to provide a new method of producing dimensionally stable light-weight wood-thermoplastic composite material of constant quality.

The novel technology is based on the concept of mixing a thermoplastic polymer selected from the group of biodegradable polymers with at least one reinforcing material selected from woody materials in the form of platy wood particles in particular under conditions of melt mixing. The mixing is carried out in an apparatus for melt processing of polymers, viz. an extruder capable of producing a compounded mixture of thermoplastic and additives, at an elevated temperature and optionally at an increased pressure. The elevated temperature corresponds to or is higher than the melt temperature of the polymer.

More specifically, the present invention is characterized by what is stated in the characterizing part of claim 1.
The invention provides considerable advantages. Thus, the method will produce light-weight dimensionally stable wood-thermoplastic composite materials composed of wood chips and a thermoplastic polycaprolactone. During the gentle manufacturing process fragile wood chips maintain their 3-D structural properties in polymer matrix resulting light, mechanically strong wood-plastic composite.
Further advantages and novel features and scope of applicability of the present invention will be set forth in the following detailed description and a number of non-limiting working examples.

### Detailed description of preferred embodiments

The material of the present invention can generally be manufactured by mixing a first component, i.e. a suitable polymer material, for example in the form of pellets, with the second component : wood particles by melt mixing in an extruder. Ideally, although not necessarily, the mixture of the first and second components prior to melting is a homogeneous mixture of the two components.

By using an extruder mixing apparatus, two hoppers, each containing one of the components of the material, can deposited the desired amount of each component in to a mixing chamber of the apparatus. Then, by way of the mixing means in the mixing apparatus, there is formed a homogeneous mixture of the first and second components prior to the formation of the composite material.
One advantage to the material being formed by such a homogeneous mixture of the components is that the forces necessary to form a substantially homogeneous material are reduced. Therefore, little or no compression force is necessary to facilitate mixing of the components in a material formation step. The importance of this factor is that, by way of the homogeneous mixture, larger particles of each component can be used which would otherwise have been destroyed when subjected to high compression forces.
The material can be applied for use after it has been recovered from the mixing device and formed into the desired shape, for example into a sheet or plate or roll or any similar planar, folded, bent or tubular structure. The material can even be formed directly on the patient.
The material mixed with an extruder can be shaped with an appropriate nozzle to the shape of, e.g. a rectangular sheet or plate which can be used directly after cutting, e.g. as a wrist splint. Such a sheet or plate whose purpose is to be later formed in to a desired shape or size is referred to herein as a blank.
A desired specific profile or shape for the splints can be manufactured with the extruder manufactured sheet or plate by optical, chemical or mechanical cutting, e.g. laser cutting, water jet cutting, eccentric pressing, or with any tool capable for producing regular shape profiles, i.e. stamping. To maintain the simplicity and cost effectiveness in the manufacturing process of the novel composite material of the present invention preferable the whole manufacturing process of the end-product is continuous.
According to an embodiment, the present method of producing a composite useful as an orthopedic material comprises the steps of;
- mixing together 10 to 100 parts, preferably 50 to 100 parts, by weight of a first component formed by a polymer selected from the group of biodegradable polymers and mixtures thereof, and
- 1 to 100 parts, preferably 10 to 50 parts, by weight of a second component formed by a reinforcing material, present in the form of platy wood particles.
The melt-mixing is carried out at a temperature sufficient for melting the thermoplastic polymer. Thus, the mixing is generally carried out at a temperature of about 50 to 190 °C, preferably about 90 to 150 °C, in particular about 100 to 130 °C, in order to achieve conditions of melt-mixing in the apparatus. The temperature can also be in the range of about 50 to 150 °C, in particularly about 100 to 140 °C.

The molten polymer mass can be subjected to tensile forces to achieve a desired orientation of the polymer and, in particular, the reinforcing particles.

Within the above general concept, the manufacturing process comprises several preferred embodiments.

Generally, a preferred embodiment of the manufacturing process of a composite material comprises the steps of compounding virgin materials with an extruder and production of uniform homogenous plate-like composite.

One particular embodiment consist of compounding wood chips and thermoplastic polymer with single screw extruder and profile manufacturing of therein produced composite by using calendaring techniques. This manufacturing method is applicable for production of composites consisting of any thermoplastic polymer having a melting point below 100 °C and wood particles having volume between 1-50 mm³.

The manufacturing process is initiated by mixing wood chips and plastic granules to uniform blend before pouring to feed hopper of the extruder. The mixing process can be carried out also by feeding of the virgin materials to the extruder directly by using separate feeding hoppers.

In a compounding process using a single screw extruder, the profile of screw has to have dimensions that allow relatively large wood chips (up to about 30 to 50 mm in one dimension) to move along the screw without crushing them. Simultaneously, appropriate dispersion between wood and polymer has to be achieved. The screw-like channel width and flight depth are selected such that formation of local pressure increases can be avoided. Similarly, the temperature of cylinder and the screw rotation speed are selected to avoid decomposition of wood chip structure by high pressure during extrusion.

The appropriate barrel temperature is generally in the range of 80 to 190 °C, preferably 100 to 150 °C, e.g. 116-135 °C, from hopper to die. The screw rotation speed is typically about 10 to 100 rpm, for example 25 to 50 rpm.

The extruder compounded composite material can be further worked upon to obtain homogeneous plates or sheets with a thickness of about 1 to 5 mm, e.g. 3.5 mm, with preselected width and length by using calendering techniques. To avoid changes in wood chip structure during calendering process, composite material can be gently folded between calendering cylinders in a plurality of phases, each consisting of a number of cycles. For example 2 phases with at least 10 cycles each are preferred.

Thus, in one embodiment, the melt mixing is carried out in an extruder and the calendering process comprises several cycles of folding, cooling and reheating steps.

The temperature of calender cylinders can be kept constant, for example at a temperature above the melting point of the thermoplastic material, to keep the composite moldable during calendering process.

Plates can be finished by sanding the edges with band sander.

In one embodiment working on polycaprolactone and wood chips, the calender cylinders were kept at 100 °C. Smooth surface to the final product were achieved by calendering the plate one time through at 100 °C.

Alternative manufacturing process for the composite plates is press method after the mixing process carried out with extruder. Pneumatic, mechanical and/or hydraulic presses are suitable for the press method of the composite. During pressing the heated plates of the press are kept at temperature

The density of composite manufactured with combination of extruder and calendaring or extruder and press vary between 600-1050, e.g. 600-850 kg/m³ depending on the weight percent of wood in material.

To maintain the simplicity and cost effectiveness in the manufacturing process of the composite of the present invention the complete manufacturing process of the end-product is preferably continuous.

During the process development it was found out that excellent results were obtained with polycaprolactones having a low to moderate melt flow index of less than 7, preferably about 0.1 to 6 g/10 min, with 2.16 kg standard die at 160 °C. By contrast, polycaprolactone polymers having a high melt flow index (MFI) (i.e. higher than the one preferred) will not give a material of desired composition. Thus, mixing of PCL having MFI values of 7g/10 minutes with 2.16 kg standard die at 160°C with wood particles using a single screw extruder the molten composite material resulted in one experiment in a material which was not self-supporting and calandering thereof was not possible. Even at temperatures close to the melting point further processing of the composite was complicated by using calandering techniques and to achieve plate-like shape for the composite heatable platen press was required.

Low viscosities of the polymers at elevated temperatures complicate the manufacturing process of the multi-component composites. For example pressing of hook-and-loop fasteners to the surface of composite at elevated temperatures tearing and creeping of the composite material can be observed. The present manufacturing method is therefore particularly well suited for the processing of highly viscous thermoplastics, such as PCL having an MFI value of 3, as a polymer component. After mixing procedure with extruder the formed mixture of wood and a PCL of the indicated MFI value can be directly used in calandering process. The creep phenomenon of the molten composite during pressing fasteners onto it can be avoided when the wood weight percentage in the composite is at least 20.

The adhesion of the composite can be improved by coating the surface of the composite with additional layer of virgin PCL or with any similarly behaving material. The coating process can be performed practically either by extrusion coating or extrusion laminating in a continuous process. In the extrusion coating a molten layer of extrudate is laid onto a composite. The temperature of the extruded molten polymer is kept below 200 °C to avoid tarnishing of the wood component and at the same time above 100 °C to guarantee reasonable flow of the polymer on the substrate. During this process of flowing, the polymer wets the entire surface evenly and smooth over uneven surface which is important contributor to adhesion.

Composite consisting of three layers of compounds (PCL-composite-PCL) can be performed by extrusion laminating. In the process two substrates (PCL) enters the nip formed by two rolls. The middle extrudate (composite) also enters the nip by traveling over each roll. The composite is therefore the center part of the resulting sandwich material.

The manufacturing of the multilayer composite consisting of composites layers having different weight percentage of wood can be performed by using calandering techniques. Two or more molten extrudates are combined to sandwich type of structures and pressed together in calander. By using similar calandering technique also padding and fasteners can be pressed to the molten composite extrudate consisting of one or multi layers.

The composite retains its shape as it cools down. It is substantially rigid but flexible so as to be supportive and comfortable. Rigidity is generally achieved when a sample heated to the above indicated softening temperature is cooled to below 50 °C, in particular to less than 45 °C, preferably less than 40°C. Typically, the composite is rigid at ambient temperature; a suitable temperature of use is about -40 to +50 °C, in particular -30 to + 40 °C.

The composite material sheet or plate can have a thickness of, generally about 0.2 to 50 mm, in particular about 1.5 to 30 mm, for example 1.5 to 20 mm. A typical thickness is about 2 to 6 mm. The length and the width of the sheet or plate can vary in the range of about 1 to 150 cm (length) and 1 to 50 cm (width), a typical length being about 10 to 60 cm and a typical width being about 5 to 20 cm.

A particular embodiment comprises producing a continuous product, collecting the product and rolling it or folding it. Such a product is then provided in the form of ribbons or tape which can be collected on a receiving roller.

As discussed above, in one embodiment, a linear product (e.g. a sheet or a plate) is produced. That laminar product can be further processed. For example it can be perforated. Another alternative is to use the linear product for making a laminate comprising at least two layers, each preferably being comprised of such linear products. Naturally, the laminate can be perforated, also.

One specific embodiment comprises the steps of providing on a laminate at least one surface layer having a reduced content of woody material. The layer can be formed by neat polymer or by a polymer merely having a reduced content of the woody material. Such a surface layer will provide improve adhesive properties for the product.

A particularly interesting embodiment comprises producing an essentially linear product, and mechanically modifying, e.g. by corrugation, the linear product so as to increase its stiffness.

The product produced by the present invention can also comprise further fillers or enforcing components. Thus, one embodiment comprises mixing the thermoplastic polymer with a first woody material and at least one second woody material, said second woody material being different from the first woody material.

A still further embodiment of producing a biodegradable linear composite material capable of being used to form an exo-skeletal device through thermal molding, comprises the steps of;
- obtaining a desired woody particle mixture, the majority of the woody particles making up the mixture being greater in size than powder,
- forming a first component comprising biodegradable polymer pellets,
- forming a second component comprising the woody particle mixture,
- mixing the first and second component into a single homogeneous mixture, and
- forming a linear composite material having a desired shape by heating the single homogeneous mixture and forming it as desired such that woody particles contained in the homogeneous mixture are not substantially degraded.

In this embodiment, obtaining a desired woody particle mixture comprises the step of taking a woody particle feed having a plurality of sized woody particles and sorting the feed to obtain the desired woody particle mixture.

As in all the above discussed embodiments, the step of sorting the woody particle feed may comprise sifting the feed through one or more meshes.

Generally, more than 70% of the second component is formed by the woody particle mixture.

The first and second components may have pellets and particles of similar size.

As in all the above discussed embodiments, in one preferred case, the desired shape is a substantially rectangular blank formed from an extruder. The desired shape may, however, be other than a rectangular blank and can be formed by optically, chemically or mechanically cutting or stamping the desired form from the composite material.

In all of the above discussed embodiments, the proportions between the components of then material can vary in a broad range. Thus, generally, 5 to 99 wt-%, for example 40 to 99 wt-%, of the material is formed by the thermoplastic polymer component and 1 to 95 wt-%, for example 1 to 60 wt-%, by the woody material.

The weight ratio of polymer-to-wood can easily be modified and the weight percent of wood, based on the total weight/volume of the composition, may vary between 1 and 70 %, preferably however in the range of 10 to 60 weight percent, or 20 to 60 weight percent, and 15 to 50 %, or 25 to 50 %, by volume.

The second component comprises a woody material having a smallest diameter of greater than 0.1 mm. As will be discussed below, there can also be other wood particles present in the second component.

The size and the shape of the wood particles may be regular or irregular. Typically, the particles have an average size (of the smallest dimension) in excess of 0.1 mm, advantageously in excess of 0.5 mm, for example in excess of 0.6 mm, suitably about 1 to 40 mm, in particular about 1.2 to 20 mm, preferably about 1.5 to 10 mm, for example about 1.5 to 7 mm. The length of the particles (longest dimension of the particles) can vary from a value of greater than 1 mm to value of about 1.8 to 200 mm, for example 3 to 21 mm.

The woody particles are platy.

Woody particles considered to be platy means that they have generally a plate-shaped character, although particles of other forms are often included in the material. The ratio of the thickness of the plate to the smaller of the width or length of the plate's edges is 1:2 to 1:50. Preferably, the woody particles include at least 10 % by weight of chip-like particles, in which the ratio of general dimension are on the order of thickness:width:length = 1:1-20:1-100, with at least one of the dimension being substantially different than another.

Based on the above, the platy particles of the present invention comprise wood particles having at least two dimensions greater than 1 mm and one greater than 0.1 mm, the average volume of the wood particles being generally at least .1 mm³, more specifically at least 1 mm³.

The wood particles may undergo some modification during the processing of the composition. For example, if blending of the first and second components is carried out with a mechanical melt processor, some of the original platy wood particles may be deformed to an extent.

The wood species can be freely selected from deciduous and coniferous wood species alike: beech, birch, alder, aspen, poplar, oak, cedar, Eucalyptus, mixed tropical hardwood, pine, spruce and larch tree for example.

Other suitable raw-materials can be used, and the woody material of the composite can also be any manufactured wood product.

The particles can be derived from wood raw-material typically by cutting or chipping of the raw-material. Wood chips of deciduous or coniferous wood species are preferred.

A particularly interesting raw-material comprises wood chips of any of the above mentioned wood species having a screened size of greater than 0.6 mm and up to about 3 mm, in particular about 1 to 2.5 mm on an average.

Substantival advantage has been found for the polymer component to be a caprolactone homopolymer or a blends of homo- or copolymers of epsilon-caprolactone having a molecular weight of above 80,000 g/mol. Specifically, polycaprolactone having a molecular weight of between 100,000 g/mol and 200,000 g/mol as been found to be advantageous both in terms of resultant properties and cost.

Before the woody particles are mixed with the thermoplastic polymer they can be surface treated, e.g. sized, with agents which modify their properties of hydrophohicity/-hydrophobicity and surface tension. Such agents may introduce functional groups on the surface of the granules to provide for covalent bonding to the matrix. Even increased hydrogen bonding or bonding due to van der Waals forces is of interest. The woody particles can also be surface treated with polymer e.g. PCL having low viscosity and molar mass values to increase holding powers between wood and PCL having high viscosity value.

The wood material can be also coated or treated with anti-rot compound e.g. vegetable oil to improve its properties against aging and impurities.

The wood material can be dehydrated to make it lighter before mixing it with polymer. The mechanical and chemical properties of wood material can be improved with heat treatment, which is known to decrease e.g. swelling and shrinkage.

In the composition according to an aspect of the present invention, the first component (the polymer) forms the matrix of the composite, whereas the microstructure of the second component in the composition in discontinuous. The particles of the second component can have random orientation or they can be arranged in a desired orientation. The desired orientation may be a predetermined orientation.

The reinforced material typically exhibits properties selected from one or several of the following:
- a density of the composition at least 5 % less than that of the polymer component (e.g. epsilon-caprolactone homopolymer) as such;
- a Young's modulus value in 3-bending test of the composition is at least 10 % higher than that of the polymer component (e.g. epsilon-caprolactone homopolymer) as such; and
- a thermal conductivity on the order of about 0.5W/m·K, at the most.

The desired composition of the second component can be achieved by sifting woody particles through one or more meshes having one or more varying qualities. The desired composition can also be accomplished by other well known techniques in the art for sorting and separating particles in to desired categories. The desired composition may be the resultant composition of one sifting or separating process. The desired composition may also be a mixture of resultant compositions from several sifting or separation processes.

In addition to wood-based powdered materials, inorganic particulates or powdered materials such as mica, silica, silica gel, calcium carbonate and other calcium salts such as tricalcium orthophosphate, carbon, clays and kaolin may be present or added.

The present method will produce a composition that can be used as a composite material. Such materials are exemplified by finger splints, wrist casts and ankle casts. Generally, the platy particles form about 30 to 70 %, preferably in excess of 40 up to about 60 %, of the total weight of the composition, for finger splints and for ankle casts about 20 to 60 %, preferably about 30 to 50 % of the total weight of the composition. There is typically a greater portion of the larger particles present in the larger casts which will reduce the total weight of the cast without impairing the strength properties thereof.

In particular, the composite material of the present invention is manufactured in to either a blank or in to a desired, specific shape or form. Ideally, the blanks and forms are linier, two dimensional and easily stackable. The blanks can be either substantially larger than the intended size to be applied to the animal or human being, herein referred to as the patient, or of substantially similar size.

In the instance when the blank is of a large size than desired, the blank can be cut with normal scissors or other conventional cutting means before application. Such a large blank is preferable in the sense that one blank may be cut in to several splints at various times according to the size required by each. Therefore, it is not necessary to store many different shapes and sizes of the material, which take up room and may be rarely used. Additionally, multiple splints may be cut from one blank in such a way as to maximize the material used and not produce a large amount of waste product.

Once the proper size and shaped piece of material is obtained, cut or selected, the material is then heated to the desired operating temperature by a heating means. Numerous heating means are known in the art, but it is preferable to uniformly heat the material to a specific desired temperature. If the temperature is too high then there is risk of discomfort or harm to the patient's skin. If the temperature is not high enough then the material will not be able to properly conform to the patient's body.

The use of the present material as a splint or cast process is described in more detail in our co-pending patent application titled "Orthopaedic Splinting System", the content of which is herewith incorporated by reference.

The following non-limiting examples illustrate the invention. In connection with the examples, reference is made to the tables below.
Table 1 is a table chart showing the densities of the test specimens manufactured by calandering at different temperatures;
Table 2 is a table chart showing the flexural strengths of the test specimens manufactured by calandering at different temperatures;
Table 3 is a table chart showing the tensile strengths of the test specimens manufactured by calandering at different temperatures; and
Table 4 is a table chart showing the densities of the test specimens with different wood weight percentage manufactured by extrusion and calandering at different temperatures.

In all the below presented examples, the polycaprolactone polymer used was a commercially available PCL homopolymer supplied under the tradename CAPA 6800 by Perstorp Ltd., Sweden). The polycaprolactone has a melt flow rate of about 3 g/10 min (measured at 150 °C and with a weight of 2.16 kg).

The wood material, if not otherwise indicated, was conventional spruce chips produced at a Finnish saw mill. In some of the examples wood particles of other wood species were used. The chips, in particular the spruce chips, were occasionally used in the form of a fraction sieved to an average size of 1 - 2.5 mm.

### Example 1

The spruce chips were dried for 4 hours in 120 °C and polymer granules were used as received. Preliminary mixing of virgin materials was carried out in a sealed plastic vessel. The mixture (200 g wood chips / 300 g PCL granules) was poured to the feed hopper connected to the Brabender single-screw extruder. The rotational speed of the extruder was set to 50 rpm and the temperatures of all four zones were fixed at 130 °C. After compounding process with the extruder, the formed composite material was heated in the oven to 125 °C to ensure its easy mouldability during calendering process. The calendering of composite mixture to a homogeneous plate was carried out in three phases which all included several cycles, folding, cooling and reheating steps. The temperature of calender cylinder was fixed at 100 °C. After calendering process the plate-like composite was cut with band-saw to size of 10 cm by 40 cm followed by one cycle calendering at 100 °C to achieve smooth surface to casting material.

### Example 2

Influence of the cylinder temperature of the calendering system to the density of composite specimens of the are shown in Table 1, flexural strengths of the specimens calendered at different temperatures are shown in Table 2 and tensile strengths of the cylinder temperature specimens are shown in Table 3. The standard deviations of measurements are included in the Tables 1 to 3. Wood weight percent in composite was fixed at 40%.

As will appear, calendering carried out at different temperatures had only a minor influence on the densities of the manufactured composite plates. Densities of the composite plates were increasing slightly at lower calandering temperatures but deviations of the measurements were varying greatly and no conclusions could be made. The flexural strengths of the corresponding materials, however, followed clear trend. At temperature of 100 °C flexural strength was ∼10 MPa and at lower temperature of 60 °C carried out calandering revealed flexural strength value of ∼ 14 MPa. The increase in bending force may seen as a result of orientation of PCL and wood particles in the composite structure.

### Example 3

Densities of the composite specimens manufactured with extruder and calendering processes. The dimensions of the wood particles are approximated values and are presented in millimeters in Table 4.

**Table 1**

| Temperature of the cylinders | Density (kg/m³) | STD |
|---|---|---|
| 60°C | 774,94 | 37,05 |
| 80°C | 764,05 | 51,46 |
| 100°C | 741,37 | 27,09 |

**Table 2**

| Temperature of the cylinders | Flexural strength (MPa) | STD |
|---|---|---|
| 60°C | 14,17 | 1,79 |
| 80°C | 13,01 | 2,22 |
| 100°C | 10,71 | 1,50 |

**Table 3**

| Temperature of the cylinders | Tensile strength (MPa) | STD |
|---|---|---|
| 60°C | 8,62 | 0,30 |
| 80°C | 6,09 | 0,85 |
| 100°C | 6,42 | 0,74 |

**Table 4**

| wood% / size | Density (kg/m³) | STD |
|---|---|---|
| Spruce 35, 1-3 mm | 669,56 | 30,81 |
| Spruce 40, 1-3 mm | 662,90 | 58,19 |
| Spruce 45, 1-3 mm | 617,35 | 26,69 |
| Spruce 50, 1-3 mm | 603,62 | 18,19 |
| polycapro lactone | 1043,53 | 24,82 |
| Aspen 40, 3-5 mm | 710,79 | 18,13 |
| Aspen 40, 1-3 mm | 733,71 | 22,72 |

### Example 5

700 g of ε-polycaprolactone CAPA 6800 and 300 g of spruce particles (dust) with average dimensions of 2 x 2 x 0.2 mm and were fed separately into a hopper of a Gimac mini twinscrew extruder. Temperatures of screw, adapter and nozzle were close 130 deg C. The composite blend was pushed out through the compounder nozzle (diameter 4 mm) and collected to the rolling belt. The composite was cooled down by pressurized air while moving on the belt. As a result a cylinder shaped homogenous mixture of wood particles and polymer was obtained.

## Claims

1. A method of producing a composite useful as splints or casts in immobilization of fractured body extremities, comprising the steps of
- mixing together a first component formed by a polymer and
- a second component formed by a reinforcing material,
**characterized by**
- mixing a thermoplastic polymer selected from the group of biodegradable polymers and mixtures thereof with a reinforcing material selected from woody materials in the form of platy wood particles having a ratio of the thickness to the smaller of the length or width of the plate's edges of between 1:2 and 1:500, and
- carrying out the mixing as melt mixing in an apparatus for melt processing of polymers, said apparatus being an extruder.

2. A method according to claim 1 wherein said platy wood particles have at least two dimensions greater than 1mm and one greater than 0.1 mm, the average volume of the platy wood particles being at least 0.1 mm³.

3. The method according to claim 1 or 2, wherein 30 to 99 parts by weight of a thermoplastic polymer is mixed together with 1 to 70 parts by weight of reinforcing particles, in particular about 40 to 80 parts by weight of thermoplastic polymer is mixed with 20 to 60 parts by weight of reinforcing particles.

4. The method according to any one of claims 1 to 3, wherein the mixing is carried out at a temperature of 50 to 190 °C, preferably 90 to 150 °C, in particular 100 to 130 °C, in order to achieve conditions of melt-mixing in the apparatus.

5. The method according to any of claims 1 to 4, wherein the thermoplastic polymer is selected from the group of epsilon-caprolactone homopolymers, blends of epsilon-caprolactone homopolymers and other biodegradable thermoplastic homopolymers, and copolymers of epsilon-caprolactone homopolymer and any thermoplastic biodegradable polymer, said thermoplastic polymer has an average molecular weight of 60,000 to 500,000 g/mol, in particular 100,000 to 200,000 g/mol.

6. The method according to any of claims 1 to 5, wherein the wood particles are orientated and aligned in a melt flow of the thermoplastic polymer before the reinforced material is shaped into an orthopedic material.

7. The method according to any of the preceding claims, wherein
- the mixing comprises compounding the thermoplastic polymer with the woody material in a melt mixing apparatus to produce a compounded melt mixture,
- providing an extrudate of the melt mixture by pultrusion of pulling-out through a mould or nozzle, and
- optionally shaping the extrudate into the form of a plate or a sheet.

8. The method according to any of claims 1 to 7, wherein the mixture of the thermoplastic polymer and the woody material is compounded in a mixing apparatus under non-crushing conditions, said mixing apparatus being selected from extruders capable of processing polymers in melt phase, in particular virgin materials are compounded in a melt mixing apparatus and shaped into a plate-like composite with calendering apparatus.

9. The method according to any of claims 1 to 8, wherein the melt mixing is carried out in an extruder and the calendering process comprises several cycles of folding, cooling and reheating steps.

10. The method according to any of claims 1 to 9, wherein the wood particles comprise chips of hardwood, softwood or a combination thereof.

11. The method according to any of claims 1 to 10, comprising producing a continuous product, collecting the product and rolling it or folding it, and optionally producing a linear product and combining at least two linear products into a laminate, and optionally providing on said laminate at least one surface layer having a reduced content of woody material.

12. The method according to any of the preceding claims, comprising mixing the thermoplastic polymer with a first woody material and at least one second woody material, said second woody material being different from the first woody material.

13. The method according to any of the preceding claims, comprising the steps of
- producing an essentially linear product and perforating said linear product, or
- producing an essentially linear product, and mechanically modifying, e.g. by corrugation, the linear product so as to increase its stiffness.

## Patentansprüche

1. Verfahren zur Herstellung eines als Schiene oder Gipsverband zur Immobilisierung von gebrochenen Körperextremitäten nützlichen Verbundstoffes, das die folgenden Schritte umfasst:
- Zusammenmischen einer ersten, durch ein Polymer gebildeten Komponente und
- einer zweiten, durch ein Verstärkungsmaterial gebildeten Komponente,
**gekennzeichnet durch**
- das Mischen eines thermoplastischen Polymers, das aus der Gruppe biologisch abbaubarer Polymere und Gemischen davon ausgewählt ist, mit einem Verstärkungsmaterial, das aus hölzernen Materialien ausgewählt ist, in Form von flachen Holzteilchen in einem Verhältnis von der Dicke zu dem kleineren aus Länge oder Breite der Kanten der Plättchen von zwischen 1:2 und 1:500 und
- das Durchführen des Mischens als Schmelzmischen in einer Vorrichtung zur Schmelzverarbeitung von Polymeren, wobei diese Vorrichtung ein Extruder ist.

2. Verfahren nach Anspruch 1, wobei die flachen Holzteilchen zumindest zwei Dimensionen von mehr als 1 mm und eine von mehr als 0,1 mm aufweisen und das mittlere Volumen der flachen Holzteilchen zumindest 0,1 mm³ beträgt.

3. Verfahren nach Anspruch 1 oder 2, wobei 30 bis 99 Gewichtsteile eines thermoplastischen Polymers mit 1 bis 70 Gewichtsteilen Verstärkungsteilchen gemischt werden, im Speziellen werden etwa 40 bis 80 Gewichtsteile thermoplastisches Polymer mit 20 bis 60 Gewichtsteilen Verstärkungsteilchen gemischt.

4. Verfahren nach einem der Ansprüche 1 bis 3, wobei das Mischen bei einer Temperatur von 50 bis 190 °C durchgeführt wird, vorzugsweise bei 90 bis 150 °C, im Speziellen bei 100 bis 130°C, um die Bedingungen des Schmelzmischens in der Vorrichtung zu erhalten.

5. Verfahren nach einem der Ansprüche 1 bis 4, wobei das thermoplastische Polymer aus der Gruppe von ε-Caprolacton-Homopolymeren, Mischungen aus ε-Caprolacton-Homopolymeren und anderen biologisch abbaubaren thermoplastischen Homopolymeren und Copolymeren aus ε-Caprolacton-Homopolymeren und einem beliebigen thermoplastischen biologisch abbaubaren Polymer ausgewählt ist, wobei das thermoplastische Polymer ein mittleres Molekulargewicht von 60.000 bis 500.000 g/mol, im Speziellen von 100.000 bis 200.000 g/mol, aufweist.

6. Verfahren nach einem der Ansprüche 1 bis 5, wobei die Holzteilchen in einem Schmelzfluss des thermoplastischen Polymers orientiert und ausgerichtet sind, bevor das Verstärkungsmaterial in ein orthopädisches Material geformt wird.

7. Verfahren nach einem der vorangegangenen Ansprüche, wobei
- das Mischen das Vermischen des thermoplastischen Polymers mit dem hölzernen Material in einer Schmelzmischvorrichtung umfasst, um ein vermischtes Schmelzgemisch herzustellen,
- das Bereitstellen eines Extrudats des Schmelzgemisches durch Pultrusion mittels Herausziehens durch eine Form oder Düse und
- gegebenenfalls Formen des Extrudats in die Form einer Platte oder eines Blattes.

8. Verfahren nach einem der Ansprüche 1 bis 7, wobei das Gemisch aus dem thermoplastischen Polymer und dem hölzernen Material in einer Mischvorrichtung unter nicht-quetschenden Bedingungen vermischt wird, wobei die Mischvorrichtung aus Extrudern ausgewählt ist, die in der Lage sind, Polymere in der Schmelzphase zu verarbeiten, im Speziellen werden neue Materialien in einer Schmelzmischvorrichtung vermischt und mittels Kalandriervorrichtung in einen plattenähnlichen Verbundstoff geformt.

9. Verfahren nach einem der Ansprüche 1 bis 8, wobei das Schmelzmischen in einem Extruder durchgeführt wird und der Kalandriervorgang mehrere Zyklen aus Falt-, Kühl- und Wiedererhitzungsschritten umfasst.

10. Verfahren nach einem der Ansprüche 1 bis 9, wobei die hölzernen Teilchen Chips aus Hartholz, Weichholz oder einer Kombination daraus umfassen.

11. Verfahren nach einem der Ansprüche 1 bis 10, umfassend das Herstellen eines kontinuierlichen Produkts, das Abnehmen des Produkts und Rollen oder Falten desselben und gegebenenfalls das Herstellen eines linearen Produkts und das Kombinieren von zumindest zwei linearen Produkten in ein Laminat sowie gegebenenfalls Bereitstellen von zumindest einer Oberflächenschicht mit einem verringerten Gehalt an hölzernem Material auf dem Laminat.

12. Verfahren nach einem der vorangegangenen Ansprüche, umfassend das Mischen des thermoplastischen Polymers mit einem ersten hölzernen Material und zumindest einem zweiten hölzernen Material, wobei das zweite hölzerne Material sich von dem ersten hölzernen Material unterscheidet.

13. Verfahren nach einem der vorangegangenen Ansprüche, das die folgenden Schritte umfasst:
- Herstellen eines im Wesentlichen linearen Produkts und Perforieren des linearen Produkts oder
- Herstellen eines im Wesentlichen linearen Produkts und mechanisches Modifizieren des linearen Produkts, z.B. durch Wellen, um dessen Steifheit zu erhöhen.

## Revendications

1. Procédé de production d'un composite utile comme attelles ou plâtres lors de l'immobilisation d'extrémités de corps fracturées, comprenant les étapes consistant à :
- mélanger ensemble un premier composant constitué d'un polymère et
- un second composant formé par un matériau de renforcement,
**caractérisé par** les étapes consistant à
- mélanger un polymère thermoplastique choisi dans le groupe constitué de polymères biodégradables et de mélanges de ceux-ci avec un matériau de renforcement choisi parmi des matériaux ligneux sous la forme de particules de bois plates ayant un rapport de l'épaisseur à la plus petite de la longueur ou de la largeur des bords de plaque entre 1:2 et 1:500, et
effectuer le mélange sous forme de mélange à l'état fondu dans un appareil de traitement à l'état fondu de polymères, ledit appareil étant une extrudeuse.

2. Procédé selon la revendication 1, dans lequel lesdites particules de bois plates ont au moins deux dimensions supérieures à 1 mm et une supérieure à 0,1 mm, le volume moyen des particules de bois plates étant d'au moins 0,1 mm³.

3. Procédé selon la revendication 1 ou 2, dans lequel 30 à 99 parties en poids d'un polymère thermoplastique sont mélangées avec 1 à 70 parties en poids de particules de renforcement, en particulier environ 40 à 80 parties en poids de polymère thermoplastique sont mélangées avec 20 à 60 parties en poids de particules de renforcement.

4. Procédé selon l'une quelconque des revendications 1 à 3, dans lequel le mélange est effectué à une température de 50 à 190°C, de préférence de 90 à 150°C, en particulier 100 à 130°C, afin d'obtenir des conditions de mélange à l'état fondu dans l'appareil.

5. Procédé selon l'une quelconque des revendications 1 à 4, dans lequel le polymère thermoplastique est choisi dans le groupe comprenant des homopolymères d'epsilon-caprolactone, des mélanges d'homopolymères d'epsilon-caprolactone et d'autres homopolymères thermoplastiques biodégradables, et des copolymères d'homopolymère d'epsilon-caprolactone et de tout polymère thermoplastique biodégradable, ledit polymère thermoplastique a une masse moléculaire moyenne de 60 000 à 500 000 g/mol, en particulier de 100 000 à 200 000 g/mol.

6. Procédé selon l'une quelconque des revendications 1 à 5, dans lequel les particules de bois sont orientées et alignées dans un flux à l'état fondu du polymère thermoplastique avant que le matériau renforcé soit mis en forme en un matériau orthopédique.

7. Procédé selon l'une quelconque des revendications précédentes, dans lequel
- le mélange comprend la composition du polymère thermoplastique avec le matériau ligneux dans un appareil de mélange à l'état fondu pour produire un mélange fondu composé,
- la fourniture d'un extrudat du mélange à l'état fondu par pultrusion de retrait à travers un moule ou une buse, et
- facultativement la mise en forme de l'extrudat sous la forme d'une plaque ou d'une feuille.

8. Procédé selon l'une quelconque des revendications 1 à 7, dans lequel le mélange du polymère thermoplastique et du matériau ligneux est composé dans un appareil de mélange dans des conditions non-écrasantes, ledit appareil de mélange étant choisi parmi des extrudeuses capables de traiter des polymères en phase fondue, en particulier des matériaux vierges sont composés dans un appareil de mélange à l'état fondu et mis en forme en un composite analogue à une plaque avec un appareil de calandrage.

9. Procédé selon l'une quelconque des revendications 1 à 8, dans lequel le mélange à l'état fondu est effectué dans une extrudeuse, et le processus de calandrage comprend plusieurs cycles d'étapes de pliage, de refroidissement et de réchauffage.

10. Procédé selon l'une quelconque des revendications 1 à 9, dans lequel les particules de bois comprennent des copeaux de bois dur, de bois tendre, ou une combinaison de ceux-ci.

11. Procédé selon l'une quelconque des revendications 1 à 10, comprenant les étapes consistant à produire un produit continu, collecter le produit et le rouler ou le plier, et éventuellement produire un produit linéaire et combiner au moins deux produits linéaires en un stratifié, et éventuellement fournir sur ledit stratifié au moins une couche de surface ayant une teneur réduite en matériau ligneux.

12. Procédé selon l'une quelconque des revendications précédentes, comprenant le mélange du polymère thermoplastique avec un premier matériau ligneux et au moins un second matériau ligneux, ledit second matériau ligneux étant différent du premier matériau ligneux.

13. Procédé selon l'une quelconque des revendications précédentes, comprenant les étapes consistant à
- produire un produit essentiellement linéaire et perforer ledit produit linéaire, ou
- produire un produit essentiellement linéaire, et modifier mécaniquement, par exemple par ondulation, le produit linéaire afin d'augmenter sa rigidité.
